# EUROPEAN PATENT APPLICATION

(11) **EP 3 647 413 A1**
(43) Date of publication of application: **06.05.2020**
(21) Application number: 18382779.9
(22) Date of filing: 31.10.2018
(51) Int. Cl.: C12N 5/0789, A61K 35/28, A61K 31/566

(54) **IMPROVEMENTS FOR PERFORMING AND FACILITATING THE RECOVERY AFTER HEMATOPOIETIC STEM CELL TRANSPLANTATION**

(71) Applicant: Centro de Investigaciones Energéticas, Medioambientales y Tecnológicas O.A., M.P. (CIEMAT), 28040 Madrid (ES); Consorcio Centro de Investigación Biomédica en Red, M.P., 28029 Madrid (ES); Fundación Instituto de Investigación Sanitaria Fundación Jiménez Díaz, 28040 Madrid (ES)
(72) Inventor: QUINTANA BUSTAMANTE, Oscar, 28040 Madrid (ES); SEGOVIA SANZ, Jose Carlos, 28040 Madrid (ES); BUEREN RONCERO, Juan A., 28040 Madrid (ES)
(74) Representative: Elzaburu S.L.P.

(57) **Abstract**

The present invention provides a method to enhance hematopoietic reconstitution and recovery after hematopoietic stem cell transplantation which is based on the administration of estrogens to the subject that has undergone the transplantation, because estrogens induced an increment in the percentage of hematopoietic cells derived from transplanted donor cells in the recipient. Additionally, estrogens increase the donor contribution in the hematopoietic stem cell compartment. A method for increasing the number of hematopoietic progenitor or stem cells in a culture is also provided, based as well in the addition of estrogens to the culture. As the obtained hematopoietic progenitor or stem cells can be also transplanted, the method increases the availability of donor cells for transplantation. Thus, the invention improves hematopoietic stem cell transplantation in patients.

## Description

### FIELD OF THE INVENTION

The present invention provides a method for enhancing hematopoietic recovery after hematopoietic stem cell transplantation. More specifically, the invention refers to a method for enhancing hematopoietic stem cell activity by estrogens.

### BACKGROUND OF THE INVENTION

Hematopoietic Stem Cells (HSCs) are a rare cell population resident in the bone marrow of adult mammals and sit atop a hierarchy of progenitors that become progressively restricted to several or a single blood lineage. HSCs are capable of self-renewal (the production of additional HSCs) and multipotent differentiation to all blood cell lineages (Orkin & Zon, 2008). HSCs are crucial in the maintenance of lifelong production of all blood cells. HSCs are highly regulated to maintain homeostasis through a delicate balance between quiescence, self-renewal and differentiation. Although HSCs divide infrequently, they are activated to self-renew in response to bone marrow injury to re-establish homeostasis (Wilson et al., 2008).

Hematopoietic stem cell transplantation (HSCT) is the most used cell therapy currently. HSCT is the recommended treatment for several life-threatening conditions such as leukemia, rare blood cell diseases, aplastic anemia, and to restore the hematopoietic system after bone marrow ablating chemotherapy or irradiation therapy. More than one million HSCT has been done worldwide so far. HSC are responsible for the lifelong production of blood cells. The special characteristics of the HSCs, such as self-renewal and multipotency, have guaranteed the success of HSCT. Among others, main bottlenecks of HSCT are HSC donor shortage and HSCT failure by infusing a reduce number of donor HSC. Different approaches have been addressed to solve these problems, such as using different sources of HSCs (bone marrow, cord blood, mobilized peripheral blood, amniotic fluid or placental cord), generating engraftable HSCs from embryonic cells, and expanding HSCs *ex vivo.* In addition, how maximizing engraftment of limiting number of HSC has been researched extensively; different HSCT modulators, either accessory cells or defined modulator molecules, are been assayed to enhance the hematopoietic reconstitution of a reduce number of donor HSC.

The term "estrogen" is applied for a group of female sex hormones, derived from cholesterol, which group comprises several compounds compounds estrone (E1), 17β-estradiol (E2, usually abbreviated as β-estradiol or simply estradiol), estriol (E3) and even the estrogen produced during pregnancy known as estetrol (E4: also known as 15α-hydroxyestriol). Estrogen is the primary female sex hormone and is responsible for controlling many cellular processes including growth, differentiation and function of the reproductive system. Estrogen acts through the genomic (nuclear-initiated steroid signaling) and non-genomic (membrane-initiated steroid signaling) signaling pathways. Additionally, both genomic and non-genomic signaling can start with the interaction of the estrogens with either Estrogen Receptor α (ESRα) or ESRβ. Consequently, the estrogen regulation of the cellular process can follow different mechanisms and maybe get different responses. Recently, it has been described that both 1β-estradiol (E2,) and estetrol (E4) signaling occurs mainly through their interaction with ESRα, although estetrol regulation is independent of non-genomic signaling (Abot et al., 2014). More specifically, according to Abot et al., (Abot et al., 2014), estetrol shows a specific profile of modulation of ESRα, different from that of β-estradiol, particularly because estetrol is not capable of triggering cell responses mediated by ESRα acting on the membrane, although it is capable of triggering other responses common to estrogens that seem to be associated with estetrol nuclear activity. Furthermore, estetrol can act as a weak estrogen antagonizing estradiol-dependent mammary gland proliferation (Gerard et al., 2015), which is an important consideration for its clinical use. Moreover, E4 has a high affinity for ESRα as compared to the ESRβ, which is different from other estrogens. Furthermore, in vivo E4 half-life is considerably longer than that of other estrogens, which compensates it apparently low potency as estrogen. Another characteristic of E4 is not bound by sex hormone-binding globulin (SHBG), in contrast to most known estrogens, which E4 serum level is representative for bio-activity and independent of SHBG levels. Lastly, another important benefit of E4 is less dependent on regulation of microsomal liver enzymes and its relative insensitivity to interact with other drugs (Coelingh Bennink, Holinka, & Diczfalusy, 2008).

Recent evidences indicate that estrogen is involved in regulating the proliferation and lineage commitment of hematopoietic stem cells (Heo et al., 2015). However, the role of estrogens in the regulation of the HSC self-renewal and multipotency needs to be clearly defined. There are few and sometime contradictory studies about the effect of the estrogen over HSC, which studies have been carried out mainly with β-estradiol, although it has been commonly accepted that it could be applicable to other estrogens. Thus, it is considered that estrogen treatment has been found to be able to increase specifically the number of vascular but not endosteal HSC in mice; however, estrogen limited the long term repopulating capacity of the HSC (Illing et al., 2012). Additionally, estrogen has been shown to promote cell cycle of HSC and multipotent progenitors (MPP) and increase erythroid differentiation in female, and effect on HSCs which is mentioned to be mediated by estrogen receptor α (ESRα) and which could be detected during pregnancy (Nakada et al., 2014).). On the contrary, tamoxifen reduces the number of MPP and short-term HSC but activate proliferation of long-term HSC (Sanchez-Aguilera et al., 2014).

Besides, estrogen can modulate HSC indirectly through acting bone formation and their mesenchymal stem cells (MSC). However, the regulation mediated by this family of hormones can be more indirect. Estrogen (β-estradiol) treatment has been described to activate MSC osteogenic differentiation and GM-CSF and IL6 secretion from these MSC, all of which improves the number of HSC by modulating their niche (Qiu_Zhao). However, this estrogen's action to regulate hematopoietic progenitors through the MSC is controversial (Illing et al., 2012). Additionally, the frequency of hematopoietic progenitors present in cord blood has been associated with the level of different hormones and growth factors, among them estriol (Savarese et al., 2007).

In spite of these evidences, the complete role of different estrogens in the regulation of hematopoietic progenitors and their underlying mechanism is unclear. So, a comprehensive study on the role of the estrogens in the HSC regulation is essential to be able to develop the clinical potential of these hormones. And, particularly, the knowledge about the activity of each estrogen (particularly, β-estradiol and estetrol) on HSCs and about the possibilities of using them in order to increase HSC number in control and/or their applicability to improve hematopoietic reconstitution in individuals who have been the subject of hematopoietic cell transplantation should be increased, in order to see if their clinical use for that purpose can be provided and to know the appropriate conditions to do it, particularly in those case where there might be a certain preference, for instance, for the enhancement of the capability of generating a particular cell lineage. Thus, it should be clarified if estrogens can be used to enhance hematopoietic recovery, the estrogens that can be used for that purpose and the conditions for that use.

The present invention provides a solution to said problem.

### SUMMARY OF THE INVENTION

The invention relates to the enhancement of recovery from hematopoietic stem cell transplantation (HSCT) by treatment with estrogens, preferably estetrol.

In a first aspect, the invention relates to an estrogen for use in enhancing hematopoietic reconstitution after hematopoietic stem cell transplantation (HSCT) or hematopoietic progenitor cell transplantation in a subject. The estrogen is preferably administered systemically and after the transplantation of hematapoietic stem cells or progenitor stem cells.

In a second aspect, the invention relates to a method for increasing the number of hematopoietic progenitor cells and/or hematopoietic stem cells in a culture, the method comprising the steps of adding an estrogen to the cell culture with the hematopoietic progenitor cells and/or hematopoietic stem cells, culturing the cells in the presence of the estrogen and, optionally, collecting the hematopoietic progenitor cells and/or hematopoietic stem cells. The cells are preferably cultured in the presence of a niche of bone marrow - mesenchymal stem cells (BM-MSCs). It can be cultured, for instance, during one week.

In third aspect, the invention relates to a hematopoietic progenitor cell or an hematopoietic stem cell or a composition comprising hematopoietic progenitor cells and/or hematopoietic stem cells for use in transplantation to a subject, wherein the cell or cells has(have) been cultured in the presence of an estrogen. The hematopoietic progenitor cell or the hematopoietic stem cell or population of hematopoietic progenitor cells and/or hematopoietic stem cells can have been obtained by applying the above method, the method of the second aspect of the invention.

For any of the aspects of the invention, the estrogen can be selected between 17β-estradiol and estetrol. Estetrol is preferred.

Also for any of the aspects of the invention, the subject that has received or is intended to receive the transplantation can be any mammal, preferably a human being.

### BRIEF DESCRIPTION OF THE FIGURES

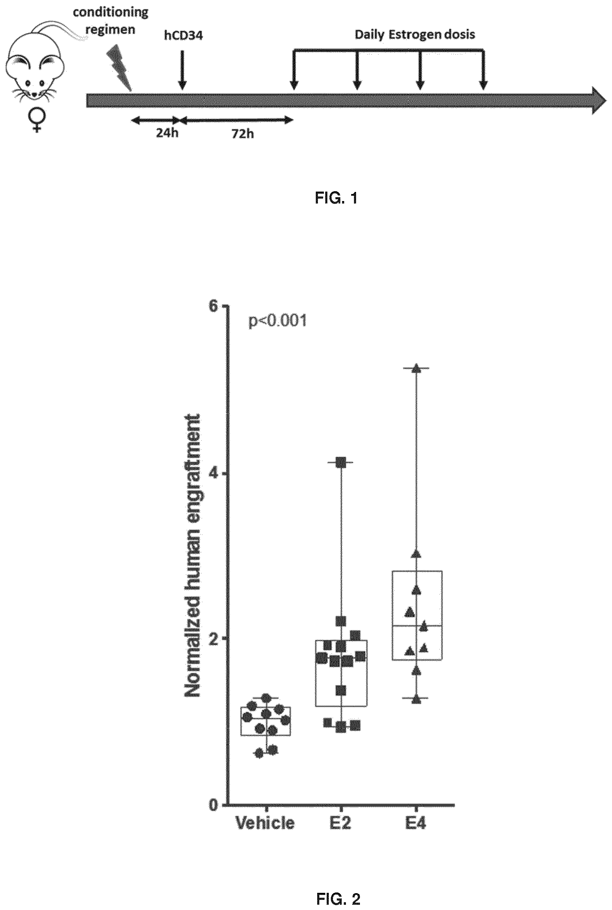

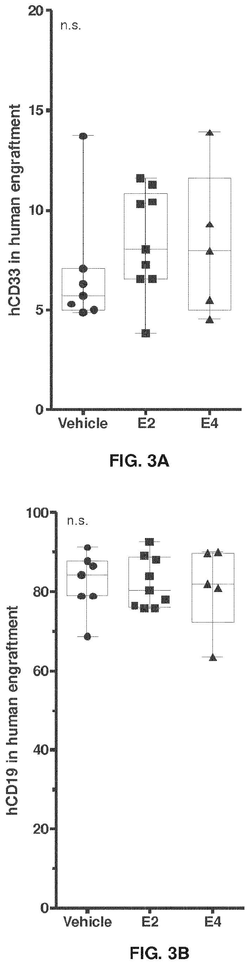

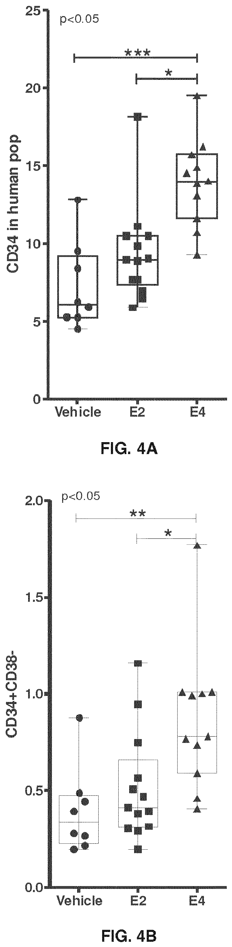

FIG. 1 presents a diagram of the protocol to test estrogen effect in xenograft mice.
FIG. 2 relates to enhance of human hematopoietic reconstitution by estrogen treatment. It represents FACS analysis of human engraftment three months post-transplant in immunodeficient male NSG mice treated for four days after hematopoietic progenitors transplant with vehicle (control) or β-estradiol (E2, 2 µg/day) or estetrol (E4, 2 µg/day). Both treatments resulted in statistically significant differences compared to controls (Kruskal-Wallis test; P<0.05, from 9 to 13 mice per group in three independent experiments).
FIGS, 3A and 3B depict multilineage reconstitution in the human hematopoietic engraftment. FIG. 4A shows percentage of myeloid cells, human hematopoietic cells expressing CD33, in human cells analyzed by FACS. FIG. 3B shows percentage of B-cells (CD19⁺ cells) in human engraftment analyzed by FACS. No treatment results in significant differences (Kruskal-Wallis test; P value no significant (n.s.., from 5 to 9 mice per group in two independent experiments)
FIGS. 4A and 4B relate to the human hematopoietic stem cell (HSC) compartment in the human hematopoietic engraftment in NSG mice. FIG. 4A and FIG. 4B. show human hematopoietic progenitors (hCD34⁺) and human HSC (hCD34⁺CD38⁻), respectively, in the human engraftment three months post-transplant. E4 treatment resulted in statistically significant differences compared to controls and E2 treated group (Kruskal-Wallis test; P<0.05, from 9 to 13 mice per group in three independent experiments).

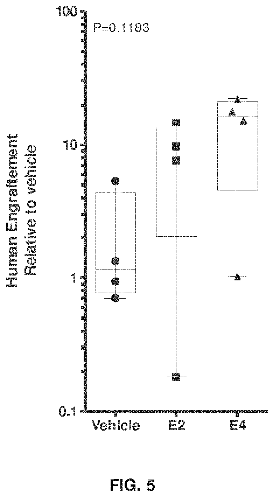

FIG. 5 indicates that estrogen treatment does not lessen long-term self-renewal capacity of human HSC. Human CD45⁺ cells were recovered from primary xenografted mice by cell sorting three montsh post-transplant. Sorted human CD45⁺ cells were transplanted in secondary female NSG. Human engraftment was analyzed 3 months post-transplant by FACS. Data were normalized to the human engraftment in the control group. No treatment results in significant differences (Kruskal-Wallis test; P=0.1183, 4 mice per group in two independent experiments).

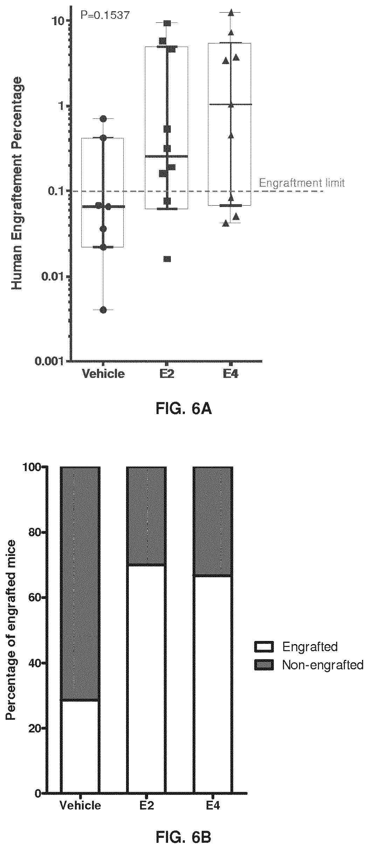

FIG. 6 depicts that estrogen treatment increases human engraftment in male immunodeficient NSG transplanted with 5x10³ CB-CD34+ and three days later, treated by vehicle E2 or E4. FIG. 6A indicates the level of human engraftment, which was raised by E4 administration.FIG. 6B shows estrogens, favored the success of the HSCT by limited HPC doses when non-engrafted animals with 0.1% or less of human hematopoietic cells in their bone marrow were considered. Around one-fourth of the mice had a human engraftment above 0.1%. However, either E2 or E4 treatment increase the proportion of engrafted mice to 70% of the animals. (Kruskal-Wallis test; P=0.1150, 7-10 mice per group).

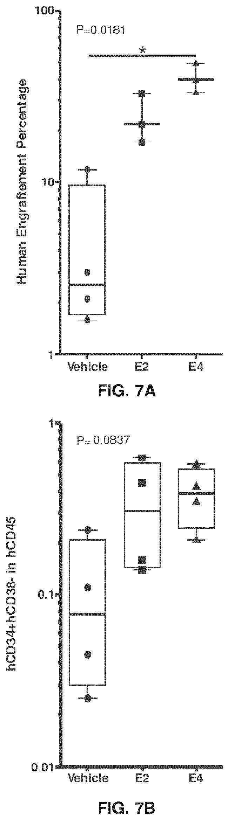

FIG. 7 shows human engraftment of low number of CB-CD34⁺ in female recipients was enhanced by estrogens. In order to know if the beneficial effect of E2 and E4 on the improvement of human hematopoietic engraftment is exclusive to male receptors, sub-lethally irradiated female NSG were transplanted with a limited number of human hematopoietic progenitors (5x10³ CB-CD34+), and three days later, were treated by vehicle E2 or E4. FIG. 7A indicates human hematopoietic reconstitution after four months. Interestingly, human engraftment in female animals was higher than in male recipients transplanted with the same reduced number of hematopoietic progenitors perhaps due to the presence of endogenous estrogens in the female animals. However, in spite of this presence of endogenous estrogens, the human engraftment in female recipients was increased after E2 and more importantly after E4 treatment. FIG 7B relates the presence of primitive hematopoietic progenitors was higher in the female mice treated with estrogens, which indicated the quality of the reconstitution. (Kruskal-Wallis test; P<0.05, 4 mice per group).

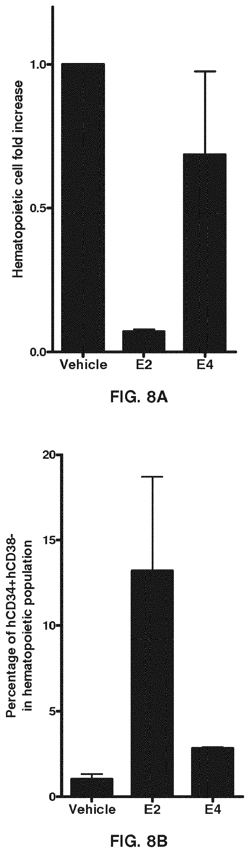

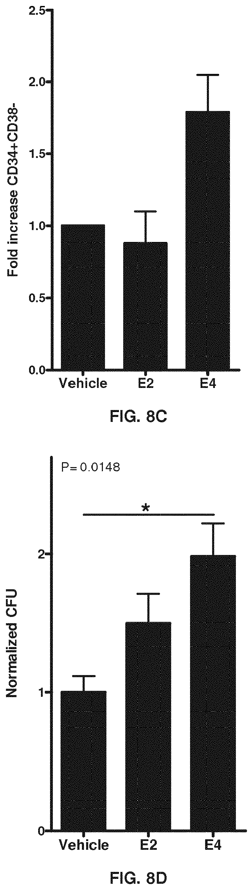

FIG. 8 shows Long-Term Culture-Initiating Cell (LTC-IC) assay. LTC-IC was established through the co-culture of human bone marrow Mesenchymal Stromal Cells (BM-MSC) and human progenitors (CB-CD34+) to research the positive effects of the estrogens in a human context, such as an in vitro human bone marrow model. LTC-ICs were treated with vehicle or 100□M of E2 or E4 for a week. FIG. 8A depicts the number of hematopoietic cells was reduced by E2, and to a minor extent by E4. FIG 8B. indicates that percentage of primitive hematopoietic progenitors was higher after estrogen treatment, mainly E2. FIG 8C. relates the total number of primitive hematopoietic progenitors did not change due to E2 treatment, but it was almost doubled after E4 treatment. FIG. 8D shows colony forming unit (CFU) analysis after 1 week of co-culture. Cells were plated in methycellulose media with specific hematopoietic cytokines to evaluate the hematopoietic progenitor content. After 14 days, colony forming units (CFU) were counted. Data were normalized to control groups. Both treatments resulted in statistically significant differences compared to controls (Kruskal-Wallis test; P<0.05, 6 replicates per group)

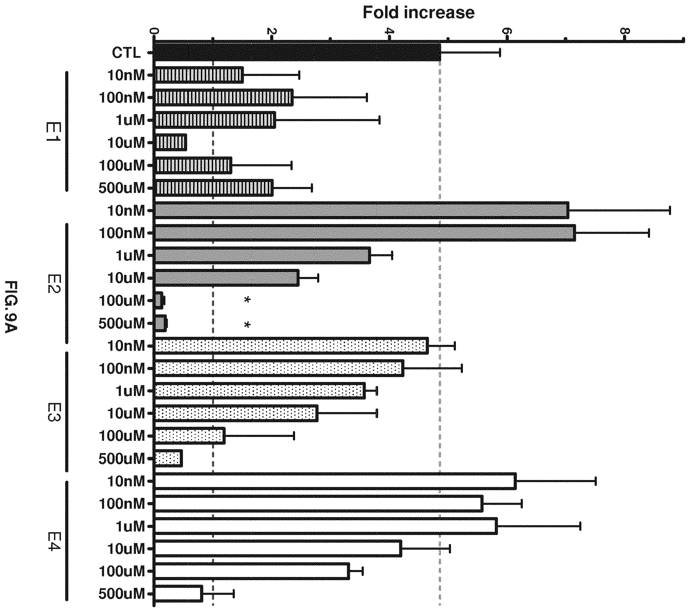

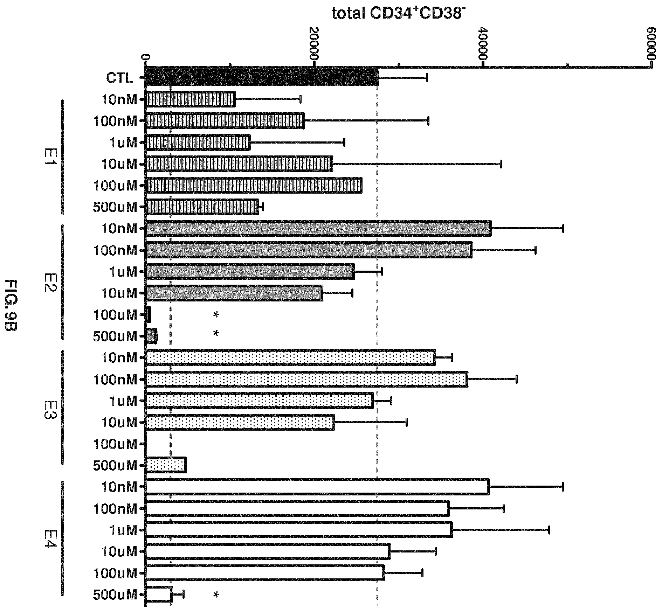

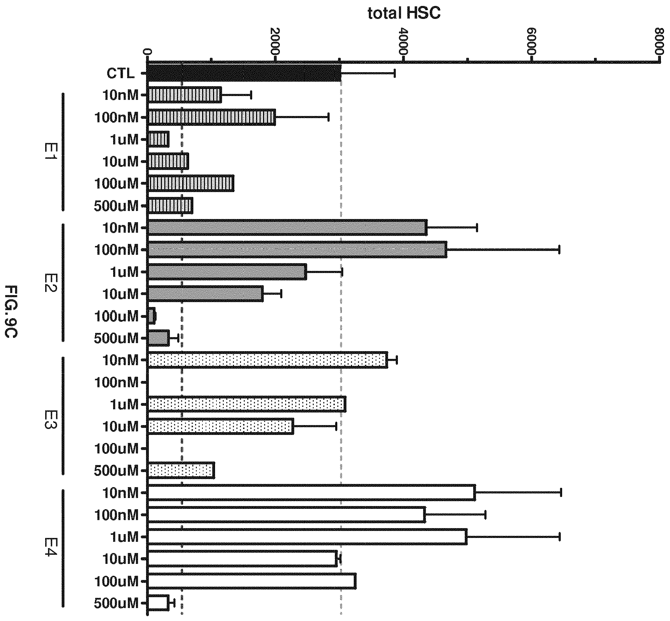

FIG. 9 relates the effect of different concentration of E1, E2, E3 and E4 in human hematopoietic progenitors. Human hematopoietic progenitors were culture in phenol red free media supplemented with 100ng/mL SCF, 100ng/ml FLT3L and 100ng/ml TPO and different concentration of E1, E2, E3 and E4 (from 10nM to 500 µM). FIG. 9A represents the fold increase of the number of hematopoietic cells after four days in culture. FIG. 9B indicates the total number of primitive hematopoietic progenitors (CD34⁺/CD38⁻) present in the culture, extrapolated from number of cells and the frequency of this population analyzed by FACS. FIG. 9C shows the total number of HSC (CD34⁺/CD38⁻/CD90⁺/CD45RA⁻) present in the culture, calculated as previously described. Grey dashed lines point the value of control group, and red dashed lines indicate the initial values at day 0. (Kruskal-Wallis test; P<0.05, data from four independent experiments).

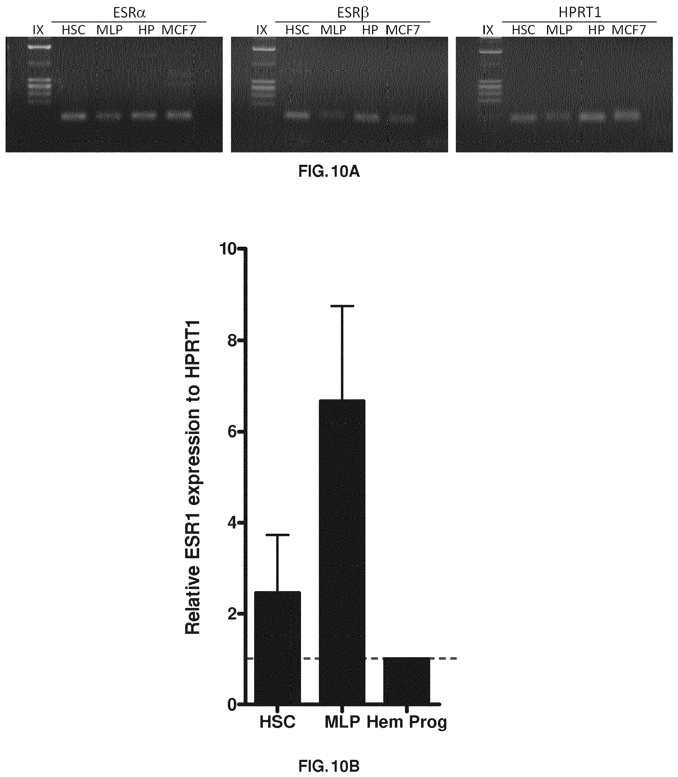

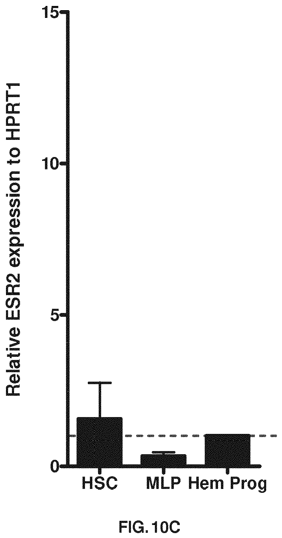

FIG. 10 indicates the expression of ESRα and ESRβ in different subset of hematopoietic progenitors. Different subpopulations of hematopoietic progenitors were sorted according their expression of CD34, CD38, CD90 and CD45RA. mRNA from HSC (CD34⁺/CD38⁻/CD90⁺/CD45RA⁻), MLP (CD34⁺/CD38⁻/CD90⁻/CD45RA⁺) and other hematopoietic progenitors (CD34⁺/CD38⁻/CD45RA⁻) was purified and retro-transcripted to cDNA, and then quantified by qRT-PCR. FIG 10A shows the presence of ESRα, ESRβ and HPRT1 transcripts in the different hematopoietic subpopulation and in a breast cancer cell line. FIG 10B. relates the relative expression of ESRα to HPRT1. FIG 10C. indicates relative expression of ESRβ to HPRT1.

### DETAILED DESCRIPTION

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

The present invention is based on the findings disclosed in the present application, where it can be seen that estrogens, and particularly 17β-estradiol (or simply estradiol) and 15α-hydroxyestriol (also known as estetrol) are capable of increasing the number of hematopoietic progenitor cells (CD34⁺) and/or hematopoietic stem cells (CD34⁺/CD38⁺) both when administered to individuals that have been the subject of hematopoietic stem cell transplantation (HSCT), thus facilitating the recovery after transplantation and the efficiency of the process, and when added to *in vitro* bone marrow-like cultures where CD34⁺ cells are present, thus facilitating the availability of hematopoietic stem cells or progenitor stem cells from a donor before using them for transplantation, because such cells previously treated with estrogens can be also effectively used for transplantation.

Thus, it can be said that the present invention provides a method to enhance hematopoietic reconstitution after HSCT, which is mainly based on the finding, disclosed in the present application, that low doses of estrogens can enhance hematopoietic engraftment cause an increment in hematopoietic reconstitution after HSCT, not only when estrogens are administered to a subject which has undergone HSCT, but also when they are administered to the cell culture where the donor cells (CD34⁺ cells) are being cultured. And it has three possible aspects:
- A estrogen for use in enhancing hematopoietic reconstitution after hematopoietic stem cell transplantation in a subject (the aspect that can be considered, more directly, also as a method to enhance hematopoietic reconstitution after hematopoietic stem cell transplantation (HSCT) in a subject, the method comprising administering an effective amount of estrogens to the subject to boost hematopoietic contribution from the transplanted hematopoietic stem cells (HSC)). The subject or individual can be any mammal which has undergone HSCT, and will be preferably a human being, particularly taken into account that the assays of the present application carried out with human cells support the applicability to human beings. The subject may suffer or have suffered from a solid tumor, myeloma, lymphoma or any blood disease requiring HSC transplantation after bone marrow ablation or myelosuppression by chemo- and/or radio-therapy. HSCT may be autologous or allogeneic to the subject. The subject may suffer anemia (sickle cell anemia, thalassemia, pyruvate kinase deficiency, Fanconi anemia or other aplastic anemia), primary or acquired immunodeficiency, an inherited genetic disease that can be cured by allogeneic hematopoietic progenitors transplantation, an inherited genetic disease that can be cured by transplantation of genetically corrected hematopoietic progenitors, or any other disease that might benefit from the transplantation of hematopoietic progenitors. HSC population may be obtained from peripheral blood, cord blood, bone marrow, amniotic fluid, placental cord or any other source of hematopoietic progenitors. HSC can be genetically modified using state of the art procedures. This aspect of the invention in particular is based on the discovery that estrogens cause an increment in hematopoietic reconstitution after HSCT. Estrogen, (mainly estetrol), administration occurs preferably by systemic route, and occurs after any myelosuppressive treatment of the subjects to avoid the HSC damage by myelosuppresive agents, since estrogen induces self-renewal of HSC, when these cells are more sensitive to these myeloablating treatments.
- A method for increasing the number of hematopoietic progenitor cells and/or hematopoietic stem cells in a culture, the method comprising the steps of adding to the cell culture an estrogen, (particularly estradiol or estetrol, and more preferably estetrol), culturing the cells in the presence of the estrogen. After a period of exposure (for instance, a week, like in Example 4), cells are collected. Preferably, the cell culture is carried out in the presence of a niche of bone marrow - mesenchymal stem cells (BM-MSCs). The results of Example 5 also show that each estrogen seem to favour the increase of the proportion of a different subset of progenitor cells, which may be useful for some particular therapeutic uses related to specific diseases. On the other hand, in accordance with the assays disclosed in the present application, the use of an estrogen, preferably estetrol, for increasing the number of hematopoietic progenitor cells and/or hematopoietic stem cells in a culture of said cells, makes not only possible to have a higher number of donor cells for transplantation, but can also reduce the proportion of hematopoietic stem cells required for HSCT. Consequently, the efficacy of HSC harvested from the subject may be improved, so the number of HSCs available for transplantation back in the subject or to another subject can be also maximized. As in the previous aspect, . HSC population may be obtained from peripheral blood, cord blood, bone marrow, amniotic fluid, placental cord or any other source of hematopoietic progenitors. HSC can be genetically modified using state of the art procedures.
- Hematopoetic stem cells or hematopoietic progenitor cells previously treated with an estrogen for use in transplantation to a subject having undergone bone marrow ablation or myelosuppression (usually by chemo- and/or radio-therapy), that is, the use of the cells obtained by the method set forth above as the second aspect of the present invention for transplantation. The subject may suffer or have suffered from a solid tumor or any blood disease requiring HSC transplantation, as commented after bone marrow ablation or myelosuppression by chemo- and/or radio-therapy. As in the first aspect of the invention, HSCT may be autologous or allogeneic to the subject. HSC population may be obtained from peripheral blood, cord blood, bone marrow, amniotic fluid, placental cord or any other source of hematopoietic progenitors. HSC can be genetically modified using state of the art procedures.

As used herein, the term "estrogens" include any substance which is capable of activating the estrogen receptors in the in vitro assays, and also include derivatives of the natural estrogen substances. For the purposes of the present invention, as the assays of the Examples of the present application have been carried out with them, the term refers particularly to β-estradiol and estetrol. For the reasons explained below, estetrol is particularly preferred for the purposes of the present invention.

The invention, and its different aspects, are based on the results of the Examples shown below.

Firstly, it is shown that estrogens modulate HSCT (Example 1), suggesting that estrogens have a role either on transplanted HSC or on the recipient bone marrow.

As shown in Example 2, low doses estrogen treatment, mainly estetrol, boosts hematopoietic engraftment and enhances the engrafted hematopoietic stem cell compartment, which ensures HSCT success.

Afterwards, to determine whether estrogens have a positive effect in both male and female recipients, female animals were transplanted with limiting cell doses and treated with estrogens. Engraftment from a limited number of human HSCs in female immunodeficient mice was also enhanced by estrogens, in particular by estetrol (Example 3). This result indicates that the estrogen treatment can have a beneficial effect in the HSCT of female and male subjects.

Moreover, as shown in Example 4, human HSCs are positively modulated in a human bone marrow-like environment, which strongly suggests the estrogen efficacy in patients.

Besides, it has been observed that estetrol is less toxic than other natural estrogens ofr hematopoietic progenitors *in vivo* and that it antagonizes partially with the non-genomic signaling of ERα in response to β-estradiol (Example 5); the latter is consistent with previous the data of previous reports (Abot et al., 2014; Gerard et al., 2015). Therefore, the modulation of HSC activity by estetrol can differ from the general effect of estrogens in hematopoietic progenitors, allowing its use in to enhance hematopoietic recovery.

This is the first time that estrogens, (mainly estetrol, but also β-estradiol), are found to act on human HSC after HSCT. As previously commented, it had been reported that female mice increase HSC self-renewal by their estrogen signaling (Nakada et al., 2014) but, on the other hand, a long treatment with estradiol seemed to increase HSC transiently, but reduced long-term repopulating activity of HSC (Illing et al., 2012). Estradiol induces the proliferation of HSC by acting on mesenchymal stem cells in vitro (Qiu, Jin, Shao, & Zhao, 2014) but, on the contrary, estrogen signaling has been shown playing a negative role on the hematopoietic progenitors and compromising HSC function (Sanchez-Aguilera et al., 2014). Contrary to the results shown in the present application, these data seemed to indicate that the estrogen action could be detrimental for HSCT. Moreover, such results had been obtained with β-estradiol and were indeed not indicative of the possible actions that might have other estrogens, like estetrol, which, in accordance with the results shown in the present application, seems to be more effective to enhance HSCT. The discrepancy between the negative effect of the estrogens (Qu, 1983) (Dieter, French, Boorman, & Luster, 1987) (Illing et al., 2012; Perry, Samuels, Bird, & Tobias, 2000; Shevde & Pike, 1996; Sontas, Dokuzeylu, Turna, & Ekici, 2009) and their beneficial application to enhance HSC activity (Hayama, Nawa, Ezaki, & Kotani, 1983; Nakada et al., 2014) can be due not only to the estrogen treatment time and doses used, but also to the differences among estrogens and the molecular pathways triggered by them in the HSC.

The results obtained with estetrol can be considered particularly surprising. As its mechanism of action had been reported to be different from the mechanism of action of other estrogens, particularly β-estradiol, it was not predictable the effect that estetrol could have on HSCs, particularly after HSCTs. Moreover, estetrol had been traditionally considered a weaker estrogen that β-estradiol or other estrogens, and, surprisingly, the assays of the Examples below show that the effects of estetrol on hematopoietic progenitors are clearly stronger than those of estradiol. Additionally, and as shown in Example 5, estetrol has a reduce toxicity in human hematopoietic progenitors, probably because it acts through a different mechanism than other estrogens (Abot et al., 2014; Gerard et al., 2015), which will facilitate the clinical use of the estetrol and makes it particularly suitable for the purposes of the present invention.

It has been described that estetrol binds to ER with less affinity than β-estradiol and with a small preference over ERβ. However, the ERα complex with estetrol was able to bind the important coactivator SRC3 as the complex with β-estradiol. Additionally, estetrol had similar solubility to β-estradiol into palmitoyl-oleoyl-phosphatidylcholine liposomes, but estetrol distribution in a bilayer (phenol at the lipid-water interface versus phenol within the hydrophobic core) was oriented predominantly toward the lipid-water interphase, whereas β-estradiol was orientated in equilibrium between the two orientations in the bilayer. These differential chemical characteristics of estetrol facilitate its special biological properties.

The method of the invention provides the following benefits: a) Patients who are considered as high risk of failed hematopoietic engraftment may be transplanted. b) Reduction of the apheresis cycles required to get an adequate harvest of HSC. c) Reduction of the dose of HSCT conditioning regimen that allows the engraftment of a specific number of HSCs. d) overall, reduction of hematopoietic transplantation failure. Estrogen, preferentially estetrol or any of its derivatives, will be administrated at low doses (0.1 mg/kg/day for 4 days) after HSCT. Blood recovery will be assessed by blood cell count and immunophenotype analyses.

Besides, it is remarkable that administration of estrogen can enlarge hematopoietic reconstitution, such that even smaller amounts of hematopoietic stem cells can then be enough in transplantation. Consequently, for example, cord blood stem cells transplantation may be applied to not only children but also adults. Additionally, estrogen administration can enhance hematopoietic reconstitution in female animals; therefore, the present invention may be applied to female subjects.

Several embodiments will now be described in detail by the following non-limiting examples.

### EXAMPLES

### - Abbreviations

In the examples below, the following abbreviations have the following meanings. Abbreviations not defined have their generally accepted meanings.
Gy = Gray
NSG = NOD.Cg-Prkdc^{scid}ll2rg^{tm1Wjl}/SzJ mouse
LTC-IC = Long-Term Culture Initiating Cells
α -MEM = alpha-modified Eagle's medium
FBS = Fetal Bovine Serum
HS = Horse Serum
PS = Penicillin/Streptomycin
hEPO = human recombinant erythropoietin
hSCF = human recombinant stem cell factor
hFLT3L = human FMS-like tyrosine kinase 3 ligand

### - Example 1. Increase of hematopoietic reconstitution after hematopoietic stem cell transplantation

Male immunodecifient NSG mice were irradiated by 2.2 Gy and transplanted by 5x10⁴ human cord-blood CD34⁺ cells twenty four hours later. 72 hours later, the transplanted animals were treated with either 2 µg of estrogen or vehicle (olive oil) daily for four days. Three-four months later, the animals were sacrificed and the human hematopoietic engraftment was analyzed by FACS. Human hematopoietic population was identified as the cells positive for hCD45-APC-Cy7 (Biolegend) and negative for mouse CD45.1-PE (Biolegend).

Estrogen or vehicle treated animals were compared. The results are shown in Table 1:

**Table 1. Percentage of human hematopoietic reconstitution**

| (mean +/- standard error of the mean and number of mice) | |
|---|---|
| **Vehicle** | 26.70 +/- 4.19 (n=10) |
| **E2** | 46.05 +/- 5.98 (n=13) |
| **E4** | 47.47 +/- 6.08 (n=9) |

Additionally, human multilineaje reconstitution of these animals was analyzed. The results are shown in Table 2:

**Table 2. Percentage in the human hematopoietic population**

| (mean +/- standard error of the mean and number of mice) | | |
|---|---|---|
| | **Myeloid** | **Lymphoid** |
| **Vehicle** | 6.86 +/- 1.18 (n=7) | 82.31 +/- 2.85 (n=7) |
| **E2** | 8.42 +/- 0.88 (n=9) | 82.26 +/- 2.14 (n=9) |
| **E4** | 8.24 +/- 1.65 (n=5) | 81.22 +/- 4.83 (n=5) |

Thus, the estrogen treated mice had approximately 80% more human hematopoietic engraftment than control animals. Moreover, there was no difference in any of the hematopoietic lineages. All together indicates that estrogen have a positive effect on the human hematopoietic reconstitution.

### - Example 2. Estrogen raises hematopoietic stem cell compartment in human hematopoietic engraftment

In order to further examine the effect of the estrogen in HSCT, hematopoietic progenitors (hCD34+) and hematopoietic stem cells (hCD34+/hCD38-) were analyzed by FACS inside of the human population in the treated NSG described in Example 1. The results are shown in Table 3.

**Table 3. Percentage in the human hematopoietic population**

| (mean +/- standard error of the mean and number of mice) | | |
|---|---|---|
| | **hematopoietic progenitors** | **hematopoietic stem cells** |
| **Vehicle** | 7.22+/-1.00 (n=8) | 0.39+/-0.08 (n=8) |
| **E2** | 9.35+/-0.86 (n=13) | 0.52+/-0.07 (n=13) |
| **E4** | 13.94+/-0.85 (n=11) | 0.87+/-0.11 (n=11) |

Moreover, to study the long-term effect in HSCT, human CD45+ cells were sorted and transplanted in secondary irradiated female NSG mice. Three months after the secondary transplant, human hematopoietic engraftment was detected in all the animals transplanted with cells from estrogen treated primary mice.

Thus, estrogen treatment resulted in a 70% increase in human hematopoietic progenitors and in a doubling in human HSC. More importantly, the treatment did not affect to the long-term human reconstitution.

### - Example 3. Estrogen raised human hematopoietic engraftment derived from the transplant of a limited CD34⁺ cell dose in male animals

To determine whether estrogen can improve hematopoietic reconstitution of low number of hematopoietic progenitors , male NSG were irradiated and transplanted with low doses of human cord blood CD34+ (5x103 hCD34+). The human engraftment was analyzed as described in Example 1. The effect of estrogen to enhance engraftment of low human progenitor doses is shown in Table 4:

**Table 4. Percentage of human hematopoietic reconstitution**

| (mean +/- standard error of the mean and number of mice) | |
|---|---|
| **Vehicle** | 0.19 +/- 0.10 (n=7) |
| **E2** | 2.12+/-1.05 (n=10) |
| **E4** | 3.20 +/- 1.43 (n=9) |

The estrogens, mainly E4, favored the success of the HSCT by limited HPC doses when non-engrafted animals with 0.1% or less of human hematopoietic cells in their bone marrow were considered. As has been shown previously, around one-fourth of the mice had a human engraftment above 0.1%. However, either E2 or E4 treatment almost tripled the proportion of engrafted mice.

To assess whether estrogen can improve hematopoietic reconstitution in female recipients, female NSG were irradiated and transplanted by a low doses of human cord blood CD34⁺ (5x10³ hCD34⁺). The human engraftment was analyzed as described in Example 1. Interestingly, human engraftment in female animals was higher than in male recipients transplanted with the same reduced number of hematopoietic progenitors, perhaps due to the presence of endogenous estrogens in the female animals. However, in spite of this presence of endogenous estrogens, the human engraftment in female recipients was increased after E2 and more importantly after E4 treatment.

**Table 5. Percentage of human hematopoietic reconstitution**

| (mean +/- standard error of the mean and number of mice) | |
|---|---|
| **Vehicle** | 4.65 +/- 2.43 (n=4) |
| **E2** | 23.90+/-4.65 (n=3) |
| **E4** | 40.80 +/- 4.58 (n=3) |

Additionally, the presence of primitive hematopoietic progenitors was higher in the female mice treated with estrogens, which indicated the quality of the reconstitution.

**Table 6. Percentage of hematopoietic stem cells (hCD34⁺/hCD38⁻) in the human hematopoietic population**

| (mean +/- standard error of the mean and number of mice) | |
|---|---|
| **Vehicle** | 0.10+/-0.05 (n=4) |
| **E2** | 0.34+/-0.12 (n=3) |
| **E4** | 0.39+/-0.08 (n=3) |

The data indicate that estrogen can also improve engraftment in recipients of limited number of hCD34⁺.

### - Example 4. Estrogen increases human progenitors in an in vitro human bone marrow-like culture

To further investigate the beneficial effect of the estrogen in human HSCT, human bone marrow- like cultures, such as LTC-IC culture, were established. 1x10⁵ human mesenchymal stromal cells (hMSC) were seeded in a p6 well and irradiated with 30 Gy next day. The following day, 5x10⁴ hCD34⁺ were added to the hMSC in LTC-IC media: 75% α-MEM without Phenol Red (Life Technologies), 12.5% Hyclone FBS (GE Healthcare), HS (Life Technologies), β-mercaptoethanol (Life Technologies), 0.5% PS and 1x10⁻⁶ M Hydrocortisone (Sigma-Aldrich). 100 µM of estrogens was added to the co-culture for a week, and then the cells were collected and the amount of human hematopoietic progenitors were evaluated in semisolid media supplement with specific hematopoietic cytokines (H4535, StemCell Technologies) plus 8 U/ml hEPO. After fourteen days, colony-forming units (CFU) were evaluated and normalized to the control:

**Table 7. Normalized human CFU**

| (mean +/- standard error of the mean and number of mice) | |
|---|---|
| **Vehicle** | 1.00 +/- 0.12 (n=6) |
| **E2** | 1.50 +/- 0.21 (n=6) |
| **E4** | 1.98 +/- 0.23 (n=6) |

Thus, estrogen treatment improved up to 98% the frequency of human hematopoietic progenitors in an in vitro human bone marrow-like culture.

### - Example 5. Estetrol showed even less toxicity than other natural estrogens for the hematopoietic cells in vitro

To assess the toxicity of the natural estrogens (E1, E2, E3 and E4) for hematopoietic progenitors, *in vitro* studies were performed. 5x10⁴ hCD34⁺ were cultured in a phenol red-free media, X-Vivo 20 (Lonza), in presence of 100 ng/ml SCF, 100 ng/ml FLT3L, 100ng/ml TPO, 0.5% PS, 1% Glutamax (Thermofisher) and different concentrations of E1, E2, E3 or E4 (from 10 nM to 500 µM). Cell proliferation and immunophenotype of different hematopoietic subsets (CD34⁺/CD38⁻, HSC [CD34⁺/CD38⁻ /CD45RA⁻/CD90⁺], Multipotent Progenitors [MPP, CD34⁺/CD38⁻/CD45RA⁻/CD90⁻], Multipotent Lymphoid Progenitors [MLP, CD34⁺/CD38⁻/CD45RA⁺] were evaluated at day 0 and four days after culture.

E1, E2 and E3 were more toxic for all the hematopoietic progenitor subpopulations than E4. On the contrary, high concentration of E4 seemed to be better tolerated for all the hematopoietic progenitor subsets, mainly the most primitive one (HSC). E4 is less toxic than other natural estrogens for hematopoietic progenitors *in vitro.*

**Table 8. Percentage of different subsets of hematopoietic progenitors after treatment with different natural estrogens**

| **Percentage of different subset of hematopoietic progenitors after E1 treatment** | | | | | | | |
|---|---|---|---|---|---|---|---|
| (mean +/- standard error) | | | | | | | |
| | **CTL** | **10nM** | **100nM** | **1µM** | **10µM** | **100µM** | **500µM** |
| **%CD34⁺/CD38⁻** | 11.45±0.17 | 12.32±2.72 | 12.91±5.83 | 10.11±2.31 | 10.54±3.34 | 11.02 | 14.80±4.62 |
| **%MLP** | 4.033±0.34 | 3.32±1.90 | 4.12±3.56 | 1,80±0.92 | 2.92±2.12 | 4.46 | 4.64±3.09 |
| **%MPP** | 4.03±0.09 | 4.69±0.88 | 4.37±1.47 | 4.48±1.50 | 3.66±0.89 | 3.86 | 5.87±1.34 |
| **%HSC** | 1.42±0.11 | 2.19±0.65 | 2.12±0.29 | 2.31 ±0.45 | 2.25±0.41 | 0.67 | 1,75±0.51 |
| | | | | | | | |
| | | | | | | | |

| **Percentage of different subset of hematopoietic progenitors after E2 treatment** | | | | | | | |
|---|---|---|---|---|---|---|---|
| (mean +/- standard error) | | | | | | | |
| | **CTL** | **10nM** | **100nM** | **1µM** | **10µM** | **100µM** | **500µM** |
| **%CD34⁺/CD38⁻** | 11.45±0.17 | 11.99±0.99 | 10.74±0.29 | 13.43±0.60 | 17.52±2.19 | 14.52±2.88 | 11.93±2.95 |
| **%MLP** | 4.033±0.34 | 4.23±0.82 | 3.72±0.31 | 3.98±0.14 | 6.79±1.96 | 1,76±0.66 | 1.01±0.24 |
| **%MPP** | 4.03±0.09 | 4.33±0.33 | 3.20±0.76 | 5.62±0.74 | 4.97±0.23 | 7.08±1.80 | 6.96±2.54 |
| **%HSC** | 1.42±0.11 | 1,52±0.24 | 4.25±2.95 | 1.59±0.15 | 1.86±0.12 | 4.50±1.91 | 3.97±2.39 |
| | | | | | | | |
| | | | | | | | |

| **Percentage of different subset of hematopoietic progenitors after E3 treatment** | | | | | | | |
|---|---|---|---|---|---|---|---|
| (mean +/- standard error) | | | | | | | |
| | **CTL** | **10nM** | **100nM** | **1µM** | **10µM** | **100µM** | **500µM** |
| **%CD34⁺/CD38⁻** | 11.45±0.17 | 14.77±0.41 | 18.28±1.30 | 14.96±0.57 | 15.89±0.62 | | 17.92±2.56 |
| **%MLP** | 4.033±0.34 | 4.94±0.70 | 6.46±0.83 | 2.72±2.38 | 5.24±0.65 | 0.15 | 4.73±1.60 |
| **%MPP** | 4.03±0.09 | 5.75±0.21 | 7.25±0.46 | 5.57±0.12 | 6.18±0.47 | 6.44 | 7.61±2.91 |
| **%HSC** | 1.42±0.11 | 1,87±0.09 | 2.28±0.03 | 4.94±2.86 | 1.95±0.13 | 4.13 | 3.70±1.51 |
| | | | | | | | |
| | | | | | | | |

| **Percentage of different subset of hematopoietic progenitors after E4 treatment** | | | | | | | |
|---|---|---|---|---|---|---|---|
| (mean +/- standard error) | | | | | | | |
| | **CTL** | **10nM** | **100nM** | **1µM** | **10µM** | **100µM** | **500µM** |
| **%CD34⁺/CD38⁻** | 11.45±0.17 | 13.35±0.87 | 12.60±0.68 | 12.08±1.22 | 13.62±0.54 | 17.26±2.24 | 10.82±2.79 |
| **%MLP** | 4.033±0.34 | 4.29±0.44 | 4.42±0.16 | 3.48±0.73 | 4.30±0.29 | 5.66±1.49 | 0.89±0.18 |
| **%MPP** | 4.03±0.09 | 4.39±0.25 | 4.35±0.56 | 4.89±0.30 | 4.57±0.35 | 3.85±0.32 | 3.11±1.20 |
| **%HSC** | 1.42±0.11 | 1.75±0.13 | 1.67±0.12 | 1,86±0.20 | 2.03±0.04 | 3.67±0.65 | 3.76±1.25 |

### - Example 6. Estrogen receptors are expressed at different levels in hematopoietic progenitors

To understand the underlying mechanism that is triggered by the different estrogens in hematopoietic progenitors, mRNA expression level of ESRα and ESRβ in the hematopoietic progenitor compartment. HSC (CD34⁺/CD38⁻/CD45RA⁻/CD90⁺), Multipotent Lymphoid Progenitors [MLP, CD34⁺/CD38⁻/CD45RA⁺] and the rest of hematopoietic progenitors (CD34⁺CD45RA⁻) were sorted; mRNA was purified by Tryzol reagent (Thermo Fisher Scientific). mRNA was retro-transcripted to cDNA by SuperScript® VILO™ cDNA Synthesis Kit (Thermo Fisher Scientific) and it was quantified by qRT-PCR using SYBR® Green (Applied Biosystems). mRNA expression level of both estrogen receptors were relative to HPRT1 expression and all the valours were normalize at ESRs of HSC subpopulation according with Pfaffl's method.

A differential expression of both ESR was identified among the different hematopoietic progenitors subset. ESRα was expressed up to four times more in MLP than in HSC. Surprisingly, ESRβ expression in HSC was double than in other progenitor. These differences might explain the different effects of the estrogens in hematopoietic progenitors.

### References

Abot, A., Fontaine, C., Buscato, M., Solinhac, R., Flouriot, G., Fabre, A., ... Arnal, J. F. (2014). The uterine and vascular actions of estetrol delineate a distinctive profile of estrogen receptor alpha modulation, uncoupling nuclear and membrane activation. EMBO Mol Med, 6(10), 1328-1346. doi:10.15252/emmm.201404112
Coelingh Bennink, H. J., Holinka, C. F., & Diczfalusy, E. (2008). Estetrol review: profile and potential clinical applications. Climacteric, 11 Suppl 1, 47-58. doi:10.1080/13697130802073425
Dieter, M. P., French, J. E., Boorman, G. A., & Luster, M. I. (1987). Metabolic characterization of mouse bone marrow cells responsive to estrogenic inhibition: hexose monophosphate shunt enzyme activity in enriched populations of mature cells and progenitor cells. J Leukoc Biol, 41(3), 212-219.
Gerard, C., Blacher, S., Communal, L., Courtin, A., Tskitishvili, E., Mestdagt, M.,... Foidart, J. M. (2015). Estetrol is a weak estrogen antagonizing estradiol-dependent mammary gland proliferation. J Endocrinol, 224(1), 85-95. doi:10.1530/JOE-14-0549
Hayama, T., Nawa, Y., Ezaki, T., & Kotani, M. (1983). Effects of estrogen on hepatic hemopoiesis in the adult mouse. Exp Hematol, 11(7), 611-617.
Heo, H. R., Chen, L., An, B., Kim, K. S., Ji, J., & Hong, S. H. (2015). Hormonal regulation of hematopoietic stem cells and their niche: a focus on estrogen. Int J Stem Cells, 8(1), 18-23. doi:10.15283/ijsc.2015.8.1.18
Illing, A., Liu, P., Ostermay, S., Schilling, A., de Haan, G., Krust, A., ... Tuckermann, J. P. (2012). Estradiol increases hematopoietic stem and progenitor cells independent of its actions on bone. Haematologica, 97(8), 1131-1135. doi:10.3324/haematol.2011.052456
Nakada, D., Oguro, H., Levi, B. P., Ryan, N., Kitano, A., Saitoh, Y., ... Morrison, S. J. (2014). Oestrogen increases haematopoietic stem-cell self-renewal in females and during pregnancy. Nature, 505(7484), 555-558. doi: 10.1038/nature12932
Orkin, S. H., & Zon, L.I. (2008). Hematopoiesis: an evolving paradigm for stem cell biology. Cell, 132(4), 631-644. doi:10.1016/j.cell.2008.01.025
Perry, M. J., Samuels, A., Bird, D., & Tobias, J. H. (2000). Effects of high-dose estrogen on murine hematopoietic bone marrow precede those on osteogenesis. Am J Physiol Endocrinol Metab, 279(5), E1159-1165.
Qiu, X., Jin, X., Shao, Z., & Zhao, X. (2014). 17beta-estradiol induces the proliferation of hematopoietic stem cells by promoting the osteogenic differentiation of mesenchymal stem cells. Tohoku J Exp Med, 233(2), 141-148.
Qu, S. Z., Yanjiao; Xu, Youheng; Tang, Huiling. (1983). Effects of estrogens on proliferation of granulocyte committed progenitor cells of mice. Hunan Yixueyuan Xuebao, 8(3).
Sanchez-Aguilera, A., Arranz, L., Martin-Perez, D., Garcia-Garcia, A., Stavropoulou, V., Kubovcakova, L., ... Mendez-Ferrer, S. (2014). Estrogen signaling selectively induces apoptosis of hematopoietic progenitors and myeloid neoplasms without harming steady-state hematopoiesis. Cell Stem Cell, 15(6), 791-804. doi:10.1016/j.stem.2014.11.002
Savarese, T. M., Strohsnitter, W. C., Low, H. P., Liu, Q., Baik, I., Okulicz, W., ... Hsieh, C. C. (2007). Correlation of umbilical cord blood hormones and growth factors with stem cell potential: implications for the prenatal origin of breast cancer hypothesis. Breast Cancer Res, 9(3), R29. doi:10.1186/bcr1674
Shevde, N. K., & Pike, J. W. (1996). Estrogen modulates the recruitment of myelopoietic cell progenitors in rat through a stromal cell-independent mechanism involving apoptosis. Blood, 87(7), 2683-2692.
Sontas, H. B., Dokuzeylu, B., Turna, O., & Ekici, H. (2009). Estrogen-induced myelotoxicity in dogs: A review. Can Vet J, 50(10), 1054-1058.
Wilson, A., Laurenti, E., Oser, G., van der Wath, R. C., Blanco-Bose, W., Jaworski, M., .. . Trumpp, A. (2008). Hematopoietic stem cells reversibly switch from dormancy to self-renewal during homeostasis and repair. Cell, 135(6), 1118-1129. doi:10.1016/j.cell.2008.10.048

## Claims

1. An estrogen for use in enhancing hematopoietic reconstitution after hematopoietic stem cell transplantation (HSCT) or hematopoietic progenitor cell transplantation in a subject.

2. The method of claim 1, wherein the estrogen is administered systemically.

3. The method of claim 1, wherein the estrogen is administered after the transplantation of hematapoietic stem cells or progenitor stem cells.

4. A method for increasing the number of hematopoietic progenitor cells and/or hematopoietic stem cells in a culture, the method comprising the steps of adding an estrogen to the cell culture with the hematopoietic progenitor cells and/or hematopoietic stem cells, culturing the cells in the presence of the estrogen and, optionally, collecting the hematopoietic progenitor cells and/or hematopoietic stem cells.

5. The method according to claim 4, wherein the cells are cultured in the presence of a niche of bone marrow - mesenchymal stem cells (BM-MSCs).

6. The method according to claim 4 or 5, wherein the cells are cultured in the presence of the estrogen during at least a week.

7. A hematopoietic progenitor cell or an hematopoietic stem cell or a composition comprising hematopoietic progenitor cells and/or hematopoietic stem cells for use in transplantation to a subject, wherein the cell or cells has(have) been cultured in the presence of an estrogen.

8. The hematopoietic progenitor cell or the hematopoietic stem cell or a population of hematopoietic progenitor cells and/or hematopoietic stem cells for use according to claim 7, wherein the hematopoietic progenitor cell or the hematopoietic stem cell or population of hematopoietic progenitor cells and/or hematopoietic stem cells have been obtained by applying the method of any one of claims 4 to 6.

9. The estrogen for use according to any one of claims 1 to 3, or the hematopoietic progenitor cell or the hematopoietic stem cell or the population of hematopoietic progenitor cells and/or hematopoietic stem cells for use according to claims 7 or 8, wherein the transplanted hematopoietic progenitor cells and/or hematopoietic stem cells are autologous or allogenic to the subject.

10. The estrogen for use according to any one of claims 1 to 3 or 9, or the hematopoietic progenitor cell or the hematopoietic stem cell or the population of hematopoietic progenitor cells and/or hematopoietic stem cells for use according to claims 7 or 8 or 9, wherein the subject is a human being.

11. The estrogen for use according to any one of claims 1 to 3 or 9 to 10, or the hematopoietic progenitor cell or the hematopoietic stem cell or the population of hematopoietic progenitor cells and/or hematopoietic stem cells for use according to claims 7 or 8 or 9 to 10, wherein the subject suffers or has suffered from a disease which is selected from the group of: solid tumor, myeloma, lymphoma any blood disease requiring HSC transplantation after bone marrow ablation or myelosuppression by chemo- and/or radio-therapy, anemia, primary or acquired immunodeficiency, an inherited genetic disease that can be cured by allogeneic hematopoietic progenitors transplantation, an inherited genetic disease that can be cured by transplantation of genetically corrected hematopoietic progenitors.

12. The estrogen for use according to any one of claims 1 to 3 or 9 to 10, or the hematopoietic progenitor cell or the hematopoietic stem cell or the population of hematopoietic progenitor cells and/or hematopoietic stem cells for use according to claims 7 or 8 or 9 to 10, wherein the subject is a candidate for bone marrow or stem cell transplantation, or a subject that has received bone marrow after ablating chemo- or radio-therapy.

13. The estrogen for use according to any one of claims 1 to 3 or 9 to 12, the method according to any one of claims 4 to 6, or the hematopoietic progenitor cell or the hematopoietic stem cell or the population of hematopoietic progenitor cells and/or hematopoietic stem cells for use according to any one of claims 7 or 8 or 9 to 12, wherein the estrogen is selected from 17β-estradiol or estetrol.

14. The estrogen for use according to any one of claims 1 to 3 or 9 to 13, the method according to any one of claims 4 to 6 or 13, or the hematopoietic progenitor cell or the hematopoietic stem cell or the population of hematopoietic progenitor cells and/or hematopoietic stem cells for use according to any one of claims 7 or 8 or 9 to 13, wherein the hematopoietic progenitor cells or the hematopoietic stem cells transplanted or to be transplanted are obtained from peripheral blood, cord blood, bone marrow, amniotic fluid, placental cord or any other hematopoietic cell source.
